# EUROPÄISCHE PATENTSCHRIFT

(11) **EP 3 373 911 B1**
(45) Veröffentlichungstag und Bekanntmachung des Hinweises auf die Patenterteilung: **26.08.2020**
(21) Anmeldenummer: 16805731.3
(22) Anmeldetag: 09.11.2016
(51) Int. Cl.: A61K 33/06, A61K 31/715, A61K 9/16, A61K 9/50, A61P 3/04, A61P 3/06

(54) **KOMBINATION VON CALCIUMSULFAT UND GUMMI ARABICUM**
COMBINATION OF CALCIUM SULFATE AND GUM ARABIC
ASSOCIATION DE SULFATE DE CALCIUM ET DE GOMME ARABIQUE

(30) Priorität: 11.11.2015 DE 102015119477
(43) Veröffentlichungstag der Anmeldung: 19.09.2018
(73) Patentinhaber: Medicocensus GmbH, 30625 Hannover (DE)
(72) Erfinder: ALBRECHT, Uwe, 31303 Burgdorf (DE)
(74) Vertreter: Schneiders & Behrendt PartmbB Rechtsanwälte - Patentanwälte
(86) Internationale Anmeldenummer: PCT/EP2016/077142
(87) Internationale Veröffentlichungsnummer: WO 2017/081079

(56) Entgegenhaltungen:
- WO-A1-2012/046142
- DE-A1- 10 028 621
- WIM CALAME ET AL: "Evaluation of satiety enhancement, including compensation, by blends of gum arabic. A methodological approach", APPETITE, ACADEMIC PRESS, NEW YORK, NY, US, Bd. 57, Nr. 2, 3. Juni 2011 (2011-06-03), Seiten 358-364, XP028273315, ISSN: 0195-6663, DOI: 10.1016/J.APPET.2011.06.005 [gefunden am 2011-06-12]
- MASAMICHI KOSEKI ET AL: "Effects of Gum Arabic and Pectin on the Emulsification, the Lipase Reaction, and the Plasma Cholesterol Level in Rats", AGRICULTURAL AND BIOLOGICAL CHEMISTRY, vol. 53, no. 12, 1 December 1989 (1989-12-01), pages 3127-3132, JP ISSN: 0002-1369, DOI: 10.1080/00021369.1989.10869844

## Beschreibung

Die Erfindung betrifft ein Mittel zur Verminderung der Fettaufnahme im menschlichen oder tierischen Körper. Das Mittel enthält Calciumsulfat und Gummi arabicum.

Es ist bekannt, dass Calciumsulfat die Aufnahme von Fetten aus der Nahrung vermindern kann und damit zur Gewichtskontrolle und -reduktion eingesetzt werden kann, siehe WO 2012/046 142 A1. Hierzu wird das Calciumsulfat in Pulverform dem einzunehmenden Nahrungsmittel zugesetzt.

Gummi arabicum wird aus dem Pflanzensaft afrikanischer Akazienarten gewonnen. Es ist ein farbloser brauner spröder Feststoff, der in warmem Wasser löslich ist. In der Galenik wird er zur Verkapselung von Arzneimitteln eingesetzt, in der Lebensmittelindustrie neben Johannisbrotkernmehl, Guarkernmehl und Alginaten als Quell- und Verdickungsmittel, Emulgator und Stabilisator verwandt. Weiterhin gilt es als effizienter Ballaststoff. In der Lebensmittelindustrie ist Gummi arabicum als Lebensmittelzusatzstoff unter der Nummer E 414 zugelassen. Es gibt keine Beschränkung der Höchstmengen, jedoch gilt das Prinzip des quantum satis, d.h. es darf nur in der gerade benötigten Menge eingesetzt werden.

Gummi arabicum ist ein natürliches Gemisch aus Polysacchariden, deren Hauptkomponente Arabinsäure ist. Es gilt als physiologisch unbedenklich und wird von Darmbakterien im Dickdarm in kurzkettige Fettsäuren umgewandelt. Es wurde nun überraschend festgestellt, dass die Kombination von Calciumsulfat und Gummi arabicum geeignet ist, die positive physiologische Wirkung des Calciumsulfats auf den Fettstoffwechsel zu verstärken. Dies gilt insbesondere dann, wenn das Calciumsulfat mit Gummi arabicum beschichtet oder darin eingehüllt ist.

Entsprechend betrifft die Erfindung ein Mittel zur therapeutischen Verwendung bei der Verminderung der Fettaufnahme im menschlichen oder tierischen Körper, enthaltend Calciumsulfat als feinkörniges Granulat eingeschlossen in Gummi arabicum, wobei die Teilchen des Granulats einzeln mit Gummi arabicum überzogen sind.

Der Verdauungsprozess von Fetten beginnt im Mund, wo langkettige Fette mit der Zungengrundlipase (pharyngale Lipase) vermengt werden. Im sauren Magenmilieu wird diese Lipase aktiviert. Es werden hier bevorzugt kurzkettige Fettsäuren aus den Triglyceriden abgespalten. Die verbleibenden ungespaltenen Fette und die Spaltprodukte der kurzkettigen Fette, also kurzkettige Fettsäuren und Glycerin, werden in den Zwölffingerdarm transportiert. Durch die in der Leber gebildete Gallenflüssigkeit, welche die Gallensalze beinhaltet, werden die Fette emulgiert und bieten dadurch den Verdauungsenzymen eine größere Angriffsfläche. Das Produkt der Bauchspeicheldrüse, die Pankreaslipase, spaltet im Wesentlichen langkettige Triglyceride. Hierdurch entstehen langkettige freie Fettsäuren. Aus mehreren Fettsäuremolekülen und Monoacylglyceriden bilden sich Micellen, die über die Dünndarmwand in den Körper aufgenommen werden.

Es wurde festgestellt, dass diese Micellen durch Calciumsulfat gebunden werden und dadurch die Aufnahme in den Körper auf physikalische Weise verhindert wird.

Die Gegenwart von Gummi arabicum fördert die Emulsionsbildung im Dünndarm und stabilisiert diese Emulsion gleichzeitig, was zu einer weiteren Vergrößerung der Oberfläche für die Bindung der Spaltprodukte führt. Dies verbessert die Bindungswirkung des Calciumsulfats an die Spaltprodukte. Es können mehr Micellen gebunden und von der Aufnahme in den Körper abgehalten werden. Somit ergänzen sich Gummi arabicum und Calciumsulfat bei der Verhinderung der Fettaufnahme durch den Körper.

Neben den stabilisierenden Eigenschaften auf die Darm gebildeten Emulsionen hat Gummi arabicum zugleich eine sättigende Wirkung - es quillt im Magen-Darm-Trakt auf und ruft dadurch ein Sättigungsgefühl hervor, was den Appetit hemmt und damit zur Gewichtskontrolle und -reduktion beiträgt.

Das erfindungsgemäße Mittel kann grundsätzlich Calciumsulfat in jeder Form enthalten, bevorzugt enthält es aber das Calciumsulfat als Dihydrat. Die im Folgenden genannten Gewichts- und Zahlenverhältnisse beziehen sich auf das Dihydrat. Wasserfreies Calciumsulfat und das Hemihydrat werden in Gegenwart von Wasser in das Dihydrat umgewandelt.

Das Gewichtsverhältnis von Calciumsulfat zu Gummi arabicum beträgt vorzugsweise 5 : 95 bis 90 : 10, insbesondere 10 : 90 bis 70 : 30 und besonders bevorzugt 30 : 70 bis 50 : 50. Für die Aktivierung des Calciumsulfat in Darm sind relativ geringe Mengen Gummi arabicum ausreichend, will man jedoch eine Quellwirkung und damit Sättigungswirkung durch das Gummi arabicum erzielen, sind größere Mengen sinnvoll.

In der Regel wird das Calciumsulfat - als Dihydrat - in fein granulierter Form eingesetzt, beispielsweise mit einer Korngröße von im Mittel weniger als 1 mm, vorzugsweise weniger als 0,5 mm. Feinere Teilchengrößen sind bevorzugt, etwa mit einer Teilchengrößenverteilung von 95 % < 0,5 mm und 5 % < 0,1 mm (90 % der Teilchen liegen im Bereich von 0,1 bis 0,5 mm).

Die Calciumsulfatteilchen werden auf herkömmliche Weise mit Gummi arabicum beschichtet. Dies geschieht beispielsweise durch Feuchtgranulierung einer Mischung aus Calciumsulfat in Pulverform, Gummi arabicum in Pulverform, der demineralisiertes Wasser sukzessive zugesetzt wird. Dabei erhält jedes Korn oder Teilchen seine eigene Ummantelung. Sprühbeschichtungsverfahren, in denen das Calciumsulfat in einer Trommel mit einer wässrigen Gummi arabicum-Lösung besprüht wird, sind ebenfalls möglich.

Das erfindungsgemäße Mittel wird kurz vor, zusammen mit oder bis zu 2 Stunden nach einem Nahrungsmittel verabreicht bzw. aufgenommen, da es seine Wirkung während des Verdauungsprozesses entfaltet. Beispielsweise kann es in Form des Granulats dem Nahrungsmittel zugesetzt werden. Vor der Nahrungsaufnahme kann es beispielsweise auch in Form einer Kapsel, Tablette oder aufgelöst in einem Getränk eingenommen werden. Die Dosis pro Mahlzeit beträgt beispielsweise 0,3 bis 5,0 g und insbesondere 0,5 bis 2,0 g. Entsprechend enthalten Kapseln, Stickpacks oder Tabletten 0,5 bis 5,0 g des Mittels.

Kapseln können beispielsweise Gelatine- oder Gummi arabicum-Kapseln sein, in die das oben beschriebene Granulat, beschichtet mit Gummi arabicum, eingefüllt ist. Tabletten können beispielsweise durch Verpressen dieses Granulats, gegebenenfalls mit einem Hilfsmittel für die Tablettierung hergestellt werden. Die Tabletten können beispielsweise auch ein Hilfsmittel enthalten, das den Zerfall in einer Flüssigkeit fördert. Das Granulat kann auch in Einzeldosen in Sachets, Stickpacks oder Siegelrandbeuteln eingebracht werden.

### Beispiel:

Mit Gummi arabicum beschichtete Calciumsulfatpartikel (Calciumssulfat Dihydrat) wurden durch Feuchtgranulation in einer Beschichtungstrommel wie folgt hergestellt.

Die Trommel wurde sukzessive mit 90 kg fein granuliertem Calciumsulfat Dihydrat, 10 kg Gummi arabicum in Pulverform und 10 l demineralisiertem Wasser beschickt und die Masse intensiv miteinander gemischt. Anschließend wurde wurde die Masse unter leichter Erwärmung getrocknet. Es ergaben sich mit Gummi arabicum beschichtete Calciumsulfatpartikel, von denen 95 % eine Größe von weniger als 0,5 mm hatten.

## Patentansprüche

1. Mittel zur therapeutischen Verwendung bei der Verminderung der Fettaufnahme im menschlichen oder tierischen Körper, enthaltend Calciumsulfat als feinkörniges Granulat eingeschlossen in Gummi arabicum, wobei die Teilchen des Granulats einzeln mit Gummi arabicum überzogen sind.

2. Mittel zur therapeutischen Verwendung nach Anspruch 1, **dadurch gekennzeichnet, dass** es das Calciumsulfat als Dihydrat enthält.

3. Mittel zur therapeutischen Verwendung nach einem der vorstehenden Ansprüche, **dadurch gekennzeichnet, dass** das Gewichtsverhältnis Calciumsulfat zu Gummi arabicum 5 : 95 bis 90 : 10 beträgt.

4. Mittel zur therapeutischen Verwendung nach Anspruch 3 mit einem Gewichtsverhältnis Calciumsulfat zu Gummi arabicum von 10 : 90 bis 70 : 30.

5. Mittel zur therapeutischen Verwendung nach Anspruch 1, **Dadurch gekennzeichnet, dass** das Granulat eine Teilchengröße hat, die zu 90 % zwischen 0,1 und 0,5 mm liegt.

6. Mittel zur therapeutischen Verwendung nach einem der Ansprüche 1 bis 5, **dadurch gekennzeichnet, dass** das Granulat in einer Kapsel enthalten ist.

7. Mittel zur therapeutischen Verwendung nach einem der Ansprüche 1 bis 5, das in einem Siegelrandbeutel enthalten ist.

8. Mittel zur therapeutischen Verwendung nach Anspruch 7, **dadurch gekennzeichnet, dass** die Kapsel eine Gelatine- oder Gummi arabicum-Kapsel ist.

9. Mittel zur therapeutischen Verwendung nach einem der Ansprüche 1 bis 5 in Tablettenform.

10. Mittel zur therapeutischen Verwendung nach einem der Ansprüche 1 bis 5, enthalten in Sachets.

## Claims

1. Means for therapeutic use in the reduction of fat absorption in the human or animal body, containing calcium sulphate as fine granulate enclosed in gum arabic, wherein the particles of the granulate are individually coated with gum arabic.

2. Means for therapeutic use according to claim 1, **characterised in that** it contains the calcium sulphate as dihydrate.

3. Means for therapeutic use according to one of the preceding claims, **characterised in that** the weight ratio of calcium sulphate to gum arabic is 5 : 95 to 90 : 10.

4. Means for therapeutic use according to claim 3, with a weight ratio of calcium sulphate to gum arabic of 10 : 90 to 70 : 30.

5. Means for therapeutic use according to claim 1, **characterised in that** the granulate has a particle size that for 90% is between 0.1 and 0.5 mm.

6. Means for therapeutic use according to any one of claims 1 to 5, **characterised in that** the granulate is contained in a capsule.

7. Means for therapeutic use according to any one of claims 1 to 5, which is contained in a sealed-rim pouch.

8. Means for therapeutic use according to claim 7, **characterised in that** the capsule is a gelatine or gum arabic capsule.

9. Means for therapeutic use according to any one of claims 1 to 5 in tablet form.

10. Means for therapeutic use according to any one of claims 1 to 5, contained in sachets.

## Revendications

1. Moyen destiné à être utilisé à des fins thérapeutiques lors de la réduction de l'absorption de graisse dans le corps humain ou animal, contenant du sulfate de calcium en tant que granulat à grains fins emprisonné dans de la gomme arabique, dans lequel les particules du granulat sont recouvertes individuellement de gomme arabique.

2. Moyen destiné à être utilisé à des fins thérapeutiques selon la revendication 1, **caractérisé en ce qu'**il contient en tant que dihydrate le sulfate de calcium.

3. Moyen destiné à être utilisé à des fins thérapeutiques selon l'une quelconque des revendications précédentes, **caractérisé en ce que** le rapport de poids entre le sulfate de calcium et la gomme arabique va de 5:95 à 90:10.

4. Moyen destiné à être utilisé à des fins thérapeutiques selon la revendication 2 avec un rapport de poids entre le sulfate de calcium et la gomme arabique allant de 10:90 à 70:30.

5. Moyen destiné à être utilisé à des fins thérapeutiques selon la revendication 1, **caractérisé en ce que** le granulat a une granulométrie, qui est comprise à 90 % entre 0,1 et 0,5 mm.

6. Moyen destiné à être utilisé à des fins thérapeutiques selon l'une quelconque des revendications 1 à 5, **caractérisé en ce que** le granulat est contenu dans une capsule.

7. Moyen destiné à être utilisé à des fins thérapeutiques selon l'une quelconque des revendications 1 à 5, qui est contenu dans un sachet à bords scellés.

8. Moyen destiné à être utilisé à des fins thérapeutiques selon la revendication 7, **caractérisé en ce que** la capsule est une capsule de gélatine ou de gomme arabique.

9. Moyen destiné à être utilisé à des fins thérapeutiques selon l'une quelconque des revendications 1 à 5 sous la forme de comprimés.

10. Moyen destiné à être utilisé à des fins thérapeutiques selon l'une quelconque des revendications 1 à 5, contenu dans des sachets.
